# EUROPEAN PATENT APPLICATION

(11) **EP 2 263 994 A1**
(43) Date of publication of application: **22.12.2010**
(21) Application number: 09723595.6
(22) Date of filing: 17.03.2009
(51) Int. Cl.: C07C 45/34, B01J 27/199, C07C 49/403, C07B 61/00

(54) **METHOD FOR PRODUCING CARBONYL COMPOUND**

(30) Priority: 19.03.2008 JP 2008071103; 16.12.2008 JP 2008319319
(71) Applicant: Sumitomo Chemical Company, Limited, Tokyo 104-8260 (JP)
(72) Inventor: NISHIMOTO, Junichi, Ibaraki-shi Osaka 567-0845 (JP); MURAKAMI, Masayoshi, Kobe-shi Hyogo 655-0049 (JP)
(74) Representative: Vossius & Partner
(86) International application number: PCT/JP2009/055123
(87) International publication number: WO 2009/116512

(57) **Abstract**

A process for producing a carbonyl compound, the process comprising reacting an olefin with molecular oxygen in the presence of a heteropoly anion, a palladium catalyst, and an iron compound, in an acetonitrile-containing aqueous solution, in the presence of an effective amount of a proton under a condition that an amount of an alkali metal in a reaction system is 1 or less g-atm per 1 mol of the heteropoly anion.

## Description

### Technical Field

The present invention relates to a process for producing a carbonyl compound.

### Background Art

As a process for producing a carbonyl compound by direct oxidation of an olefine, the Wacker process, which uses a PdCl₂-CuCl₂ catalyst, has been known for a long time. However, there have been problems with corrosion of equipment by chlorine, by-products containing chlorine compounds, and the like in the Wacker process. Moreover, there are problems that the reaction rate markedly decreases as the carbon number of an olefin material increases and that the reactivity of an internal olefin is low, and the process thus has not been used industrially except in the manufacture of lower carbonyl compounds such as acetaldehyde, acetone, and the like. As a method to resolve these problems, Patent Document 1 discloses a method of carrying out a reaction adding a redox metal under the presence of palladium and a heteropoly acid.

Patent Document 1: Japanese Unexamined Patent Application Publication (Translation of PCT Application) No. 63-500923

### Disclosure of the Invention

### Problem which the invention is to solve

However, the method described in Patent Document 1 cannot satisfy the needs from the perspective of productivity because the activity per a unit amount of Pd is insufficient for oxidation of a cyclic olefin.

### Means for solving the problem

The present invention relates to a process for producing a carbonyl compound, the process comprising reacting an olefin with molecular oxygen in the presence of a heteropoly anion, palladium, and an iron compound, in an acetonitrile-containing aqueous solution, under a condition that an amount of an alkali metal in a reaction system is 1 or less g-atm per 1 mol of the heteropoly anion.

### Effect of the Invention

According to the present invention, a corresponding carbonyl compound can be efficiently produced from an olefin by increasing the activity per a unit amount of Pd.

### Best Modes for Carrying Out the Invention

The palladium sources that can be used as a palladium catalyst in the present invention include, for example, palladium metals, palladium compounds, and the mixtures thereof. Specific examples of the palladium compounds include, for example, organic acid salts of palladium, oxyacid salts of palladium, palladium oxide, and palladium sulfide. In addition, the examples include these salts and oxides, organic or inorganic complexes of the sulfide, and the mixtures thereof.

Examples of the organic acid salts of palladium include, for example, palladium acetate and palladium cyanide. Examples of the oxyacid salts of palladium include, for example, palladium nitrate and palladium sulfate. Examples of these salts and oxides, and organic or inorganic complexes of the sulfide include, for example, tetraaminepalladium (II) nitrate, bis(acetylacetonato)palladium, and the like. Among them, the organic acid salts of palladium or the oxyacid salts of palladium are preferred, and palladium acetate is more preferred. When cyclohexene is oxidized, in particular, a palladium source isn't chloride, but is desirable to be devoid of chlorines.

In the present invention, the reaction is carried out under a condition in which an amount of an alkali metal in a reaction system is 1 or less g-atm per 1 mol of the heteropoly anion. Accordingly, if the concentration of the alkali metal is kept within this range, a heteropolyacid salt of the alkali-metal type can be used. Although a method of preparing the acid salt is not particularly limited, any composition of the heteropolyacid salt can be synthesized, for example, by preparing an aqueous solution containing the heteropoly acid and the predetermined amount of the alkali metal source, and by evaporating the solvent to dryness. Preferably, those in which all the counter ion is a proton are used.

The heteropoly anion that is used in the present invention is preferably a heteropoly anion having at least one element selected from the group consisting of P, Si, V, Mo, and W, and a more preferred heteropoly anion is a heteropoly anion having at least one element selected from the group consisting of P, V, Mo, and W.

Examples of typical composition of a heteropoly anion constituting the acidic heteropoly acid and the heteropolyacid salt include those having the composition formula of the following (1A) and (1B).
XM₁₂O₄₀ (1A) wherein X is P or Si, and M represents at least one element selected from the group consisting of Mo, V, and W; or

X₂M₁₈O₆₂ (1B)

wherein X is P or Si, and M represents at least one element selected from the group consisting of Mo, V, and W.

Examples of the heteropoly anions of the heteropoly acid having such composition include heteropoly anions (e.g., phosphomolybdic acid, phosphotungstic acid, silicomolybdic acid, silicotungstic acid, phosphomolybdotungstic acid, phosphovanadotungstic acid, phosphovanadomolybdic acid, etc.). Among them, the heteropoly acids containing vanadium are preferably used, and phosphovanadotungstic acid or phosphovanadomolybdic acid is particularly preferred. More specifically, H₄PV₁Mo₁₁O₄₀, H₅PV₂Mo₁₀O₄₀, H₆PV₃Mo₉O₄₀, and H₇PV₄Mo₈O₄₀ are used as a particularly preferred heteropoly acid.

Although a preferred additive amount of the heteropoly acid or acid salt of the heteropoly acid depends on the type of the heteropoly acid, in many cases, the range of the concentration in an acetonitrile-containing aqueous solution is preferably 0.1 mmol/L to 100 mmol/L, and more preferably 1 mmol/L to 50 mmol/L. Additionally, the additive amount of the heteropoly acid or acid salt of the heteropoly acid is usually 50 to 0.1 mol per 1 mol of palladium, preferably 20 to 0.5 mol per 1 mol of palladium, and more preferably 1 to 10 mol per 1 mol of palladium.

The oxidation reaction is carried out under the presence of the effective amount of a proton, and can also be carried out by separately adding a protonic acid other than the heteropoly acid or the acid salt thereof. The protonic acids that may separately be added other than the heteropoly acid or the acid salt thereof include inorganic acids, organic acids, and solid acids. The inorganic acids include hydrochloric acid, binary acid (hydracid) such as hydrofluoric acid, sulfuric acid, and oxo acid (oxyacid) such as nitric acid. Examples of the organic acids include, for example, formic acid, aliphatic carboxylic acid (e.g., acetic acid), alicyclic carboxylic acid (e.g., cyclohexanecarboxylic acid), aromatic carboxylic acid (e.g., benzoic acid), sulfonic acid (e.g., p-toluenesulfonic acid), and the like. Examples of the solid protonic acids include, for example, ion exchange resin (e.g., sulfonic acid-type ion exchange resin, etc.), acidic zeolite and the like, and sulfated zirconia.

The additive amount of the protonic acid other than the heteropoly acid is preferably 10 or less g-atm per 1 mol of the heteropoly anion. Besides, more preferred is to supply the effective amount of the proton by adding the heteropoly acid or the acid salt thereof.

Although a known compound, but is not limited to, can be used as an iron compound in the present invention, the specific compounds include, for example, iron sulfate, iron alum (ammonium iron sulfate), iron nitrate, inorganic salts such as iron phosphate, iron citrate, organic acid salts such as iron acetate, iron phthalocyanine, complexes such as iron acetylacetonato, iron oxide, and the like. Among them, the inorganic salts of iron are preferably used, and iron sulfate or iron alum is suitable. The additive amount of the preferred iron compound is 0.01 to 100 mol per 1 mol of the heteropoly acid, and more preferably 0.1 to 50 mol per 1 mol of the heteropoly acid.

In the present invention, the reaction is performed in an acetonitrile-containing aqueous solution. Since the preferred ratio by weight of acetonitrile/water depends on the type of the heteropoly acid used and on the reaction conditions, the ratio cannot be uniformly defined, but the acetonitrile is preferably in an amount of 4.8 to 0.1 parts by weight per 1 part by weight of water, and more preferably 3 to 0.2 parts by weight per 1 part by weight of water. The above range of the usage of acetonitrile is suitable for the case using, for example, phosphovanadomolybdic acid and for other cases.

The olefin that is used in the present invention is not limited, but a cyclic ketone can be obtained efficiently by oxidation of a cyclic olefin in particular. Examples of the cyclic olefin include a cyclic olefin having the carbon number of 4 to 20. These include, for example, cyclobutene, cyclopentene, cyclohexene, cycloheptene, cyclooctene, cyclodecene, cyclododecene, cyclooctadecene, and the like. The cycloolefin that is more preferably used is cyclohexene, and cyclohexanone is produced efficiently from cyclohexene.

Pure oxygen or air can be used as molecular oxygen, and the oxygen may be used as a gas containing molecular oxygen by diluting these gases with an inert gas such as nitrogen, helium, or the like. The amount of oxygen used is usually adjusted by the pressure of the oxygen-containing gas that is injected into a reaction system, and the range of preferably 0.01 to 10 MPa and more preferably 0.05 to 5 MPa as oxygen partial pressure is set. This reaction gas, as a total volume, may be injected before reaction, or the reaction may be carried out by continuously supplying the gas, such as by blowing in the system during the reaction, and the like.

The reaction is usually performed in a pressure range of 0.01 to 10 MPa (gauge pressure), preferably 0.05 to 7 MPa (gauge pressure), and more preferably 0.1 to 5 MPa (gauge pressure). The oxidation reaction is usually performed in a temperature range of 0 to 200°C, preferably 10 to 150°C, and more preferably 30 to 100°C.

The reaction solution or reaction gas, both of which contain a product, is collected to isolate a carbonyl compound such as a desired ketone corresponding to an olefin and the like. The produced ketone compound can be separated usually by distillation, phase separation, and the like. Examples of the ketones include, for example, cyclopentanone, cyclohexanone, cyclododecanone, and the like. The reaction can be performed by a batch, a semibatch, a continuous process or the combinations thereof.

### Examples

Hereinafter, the present invention will be further illustrated in detail by the Examples, but is not limited to the following Examples.

### Example 1

The following mixture was placed in a 120-ml autoclave, and was reacted at 323 K for 2 hours under 2 MPa of air and 3 MPa of nitrogen (0.42 MPa of oxygen partial pressure, 4.58 MPa of nitrogen partial pressure) during stirring with a stirring bar. The obtained reaction mass was analyzed by gas chromatography. The results are shown in Table 1.

### (Mixture)

Cyclohexene: 1.6 g (20 mmol),
Solvent: acetonitrile/water (3.0 ml/2.0 ml),
Pd(OAc)₂: 4mg(0.02 mmol),
H₇PV₄Mo₈O₄₀ (manufactured by NIPPON INORGANIC COLOUR & CHEMICAL CO., LTD.): 120 mg (0.06 mmol),
Iron alum(FeNH₄(SO₄)₂·12H₂O, KANTO CHEMICAL CO., INC.): 58 mg, (0.12 mmol).

### Example 2

The reaction was performed in the same manner as Example 1 except that acetonitrile/water (2.0 ml/3.0 ml) was used as a solvent. The results are shown in Table 1.

### Example 3

The following mixture was placed in a 120-ml autoclave, and was reacted at 323 K for 2 hours under 2 MPa of air and 3 MPa of nitrogen (0.42 MPa of oxygen partial pressure, 4.58 MPa of nitrogen partial pressure) during stirring with a stirring bar. The obtained reaction mass was analyzed by gas chromatography. The results are shown in Table 1.

### (Mixture)

Cyclohexene: 0.32 g (4 mmol),
Solvent: acetonitrile/water (3.0 ml/2.0 ml),
Pd(OAc)₂: 4 mg (0.02 mmol),
H₃PMo₁₂O₄₀ (NIPPON INORGANIC COLOUR & CHEMICAL CO., LTD.): 240 mg,
Iron sulfate (Fe₂(SO₄)₃·nH₂O, KANTO CHEMICAL CO., INC.): 58 mg (0.12 mmol).

### Comparative Example 1

The reaction was performed in the same manner as Example 1 except not using iron alum. The results are shown in Table 1.

### Comparative Example 2

According to a method disclosed in Patent Document 1, K₅H₄PMo₆V₆Mo₄₀ was prepared as follows. Specifically, 7.32 g of sodium metavanadate was dissolved into 38 ml of distilled water, and the mixture was kept at 90°C. In addition to this, 8.07 g of sodium molybdate was added to 12 ml of distilled water, and the mixture was heated to 90°C. Then, the above-prepared aqueous sodium metavanadate solution was added thereto. To this mixture was added 5 ml of 85% phosphoric acid. After the mixture was cooled, and stirred while adding 8 g of potassium nitrate, and the solid was then filtrated. The solid was recrystallized from 0.25 M of H₂SO₄. The resultant solid was subjected to elemental analysis and was found to be K₅H₄PMo₆V₆O₄₀.

The oxidation reaction of cyclohexene was performed in the same manner as Example 1 except that 100 mg (0.06 mmol) of K₅H₄PMo₆V₆O₄₀ thus prepared was used and 8.7 mg of sulfuric acid was further added.

### Comparative Example 3

By using the mixture of the catalyst described in Patent Document 1, the oxidation reaction of cyclohexene was performed as follows. Specifically, to acetonitrile/water (1.3 ml/3.8 ml) was added 8 mg of Pd (NO₃)₂, 160 mg (0.09 mmol) of K₅H₄PMo₆V₆O₄₀ prepared in Comparative Example 2, and 120 mg of Cu(NO₃)₂·3H₂O, and further added 7.7 mg (0.08 mmol) of sulfuric acid. To this was added 210 mg (2.6 mmol) of cyclohexene. The mixture was placed in a 120-ml autoclave, and was reacted at 323 K for 2 hours under 2 MPa of air and 3 MPa of nitrogen (0.42 MPa of oxygen partial pressure, 4.58 MPa of nitrogen partial pressure) during stirring with a stirring bar. The obtained reaction mass was analyzed by gas chromatography. The results are shown in Table 1.

### Comparative Example 4

The oxidation reaction of cyclohexene was performed in the same manner as Example 3 except not using iron sulfate. The results are shown in Table 1.

**[Table 1]**

| | Heteropoly acid | Iron compound | Acetonitrile/Water* | Protonic acid | Conversion rate | Selectivity | TOF (h⁻¹) | Acetamide/ Cyclohexanone (mol%) |
|---|---|---|---|---|---|---|---|---|
| Example 1 | H₇PV₄Mo₈O₄₀ | Iron alum | 1.2 | - | 38 | 92 | 196 | 7.5 |
| Example 2 | H₇PV₄Mo₈O₄₀ | Iron alum | 0.5 | - | 20 | 91 | 100 | 11 |
| Example 3 | H₃PMo₁₂O₄₀ | Iron sulfate | 1.2 | - | 61 | 95 | 69 | 0.8 |
| Comparative Example 1 | H₇PV₄Mo₈O₄₀ | - | 1.2 | - | 12 | 81 | 68 | 5.6 |
| Comparative Example 2 | K₅H₄PMo₆V₆O₄₀ | Iron alum | 1.2 | Sulfuric acid | 4 | 83 | 23 | 31 |
| Comparative Example 3 | K₅H₄PMo₆V₆O₄₀ | Cu(NO₃)₂ | 0.4 | Sulfuric acid | 23 | 73 | 7 | 119 |
| Comparative Example 4 | H₃PMo₁₂O₄₀ | - | 1.2 | - | 12 | 92 | 14 | 0.0 |

| | | | | | | | | |
|---|---|---|---|---|---|---|---|---|
| * Ratio by weight | | | | | | | | |

In Table 1, all the experimental results show that the conversion rate represents the conversion rate of cyclohexene, that the selectivity is the ratio of generated cyclohexanone to converted cyclohexene, and that TOF (h⁻¹) means (number of moles generated for cyclohexanone)/(number of moles of Pd)/(reaction time).

### Industrial Applicability

The present invention is applicable as an industrial process for producing a corresponding carbonyl compound from an olefin.

## Claims

1. A process for producing a carbonyl compound, the process comprising reacting an olefin with molecular oxygen in the presence of a heteropoly anion, a palladium catalyst, and an iron compound, in an acetonitrile-containing aqueous solution, in the presence of an effective amount of a proton under a condition that an amount of an alkali metal in a reaction system is 1 or less g-atm per 1 mol of the heteropoly anion.

2. The process according to claim 1, wherein the olefin is a cyclic olefin and the carbonyl compound is a cyclic ketone.

3. The process according to claim 1, wherein the olefin is cyclohexene and the carbonyl compound is cyclohexanone.

4. The process according to any of claims 1 to 3, wherein the heteropoly anion contains vanadium.

5. The process according to any of claims 1 to 4, wherein an amount of acetonitrile contained in the acetonitrile-containing aqueous solution is 4.8 to 0.1 parts by weight per 1 part by weight of water contained in the aqueous solution.
